# EUROPEAN PATENT APPLICATION

(11) **EP 2 842 952 A1**
(43) Date of publication of application: **04.03.2015**
(21) Application number: 13780982.8
(22) Date of filing: 24.04.2013
(51) Int. Cl.: C07D 337/14, A61K 31/38, A61P 29/00

(54) **METHOD FOR PRODUCING ZALTOPROFEN AND DERIVATIVE THEREOF**

(30) Priority: 24.04.2012 JP 2012098736
(71) Applicant: Nippon Chemiphar Co., Ltd., Tokyo 101-0032 (JP)
(72) Inventor: TENDO, Atsushi, Misato-shi Saitama 341-0005 (JP)
(74) Representative: Godemeyer Blum Lenze Patentanwälte Partnerschaft mbB
(86) International application number: PCT/JP2013/061974
(87) International publication number: WO 2013/161842

(57) **Abstract**

The present invention relates to a method for preparing zaltoprofen or a zaltoprofen derivative by a Friedel-Crafts reaction type intramolecular cyclization reaction in an industrial scale at an industrially acceptable yield and a purity sufficient for use as an active ingredient of medicaments in a simple and economical manner, without generating a detectable amount of impurities such as dimmers, by using polyphosphoric acid in an amount of 6.5 times or less of amount of a starting material compound before cyclization. More specifically, the present invention relates to such a method for preparing zaltoprofen or a zaltoprofen derivative as mentioned above, which comprises cyclizing a starting material compound before cyclization by an intramolecular cyclization reaction thereof in the presence of one or more kinds of organic solvents selected from the group consisting of 1,1,2-trichloroethene, methylcyclohexane, cyclohexane, n-heptane and the like.

## Description

### Technical Field

The present invention relates to a novel method for preparing zaltoprofen and a derivative thereof.

### Background Art

It is known that the compounds represented by the following formula (A) (in the formula, R¹ represents a lower alkyl group) have superior anti-inflammatory activity and analgesic action as a nonsteroidal analgesic antiphlogistic agent (Japanese Patent Publication (Kokoku) No. 61-7199, Japanese Patent Unexamined Publication (Kokai) No. 2005-289949).

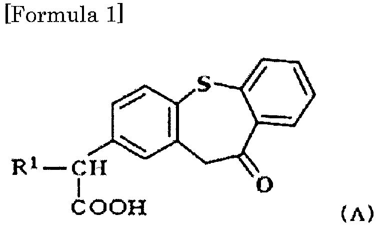

The compounds of the aforementioned formula (A) can be obtained by using a compound represented by the following formula (B) (in the formula, R¹ and R² independently represent a lower alkyl group): 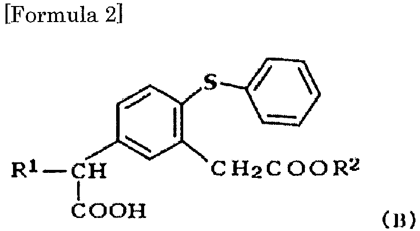 as a starting material, and subjecting the material to a Friedel-Crafts reaction type intramolecular cyclization reaction (Japanese Patent Publication No. 1-29793, Japanese Patent Unexamined Publication No. 2005-289949).

Among the compounds of the aforementioned formula (A), in particular, the compound wherein R¹ is CH₃, 2-(10,11-dihydro-10-oxodibenzo[b,f]thiepin-2-yl)propionic acid, which is represented by the following formula (III), is referred to as zaltoprofen as a generic name, and has been widely used as a nonsteroidal analgesic antiphlogistic agent.

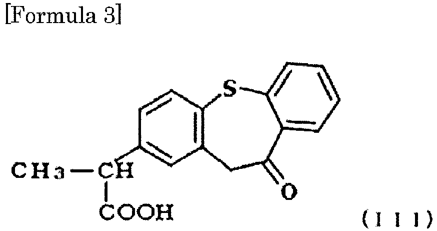

As for the method for preparing zaltoprofen, the compound can be prepared by cyclizing only one of the two carboxylic acid groups of 5-(1-carboxyethyl)-2-phenylthiophenylacetic acid represented by the following formula (IV): 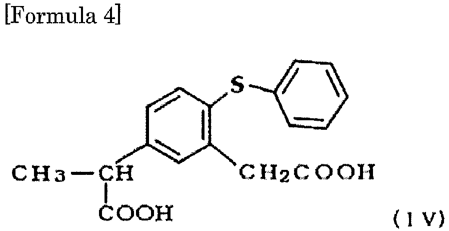 by a Friedel-Crafts reaction type intramolecular cyclization reaction in the absence of organic solvent (Japanese Patent Publication No. 1-29793 (Example 3)).

However, as for the temperature for the intramolecular cyclization reaction, it has been reported that "a temperature higher than 70°C is not advantageous because stirring at a high temperature causes side reactions" (Japanese Patent Unexamined Publication No. 57-171991), whereas it has also been reported that "the reaction can be preferably carried out by heating to a temperature near the boiling point of an organic solvent used" (Japanese Patent No. 4603276). Therefore, it has remained unknown whether a temperature near the boiling point is preferred, or whether a low temperature is preferred.

Although the target compound is obtained in a high isolation yield as high as 84.8% by the method of Japanese Patent Publication No. 01-29793, it is necessary to use polyphosphoric acid for the reaction in a large excess amount with reference to the starting material compound (for example, 20-fold amount by weight of the starting material compound) (Japanese Patent Publication No. 01-29793, Example 2). Since the polyphosphoric acid used as a condensing agent cannot be recovered and reused, the method requires high cost for disposal of the reaction wastes. Moreover, polyphosphoric acid is a very strong acid, and the phosphoric acid waste fluid produced from the decomposition treatment is one of the causes of marine pollution. Therefore, the influence of the disposal process thereof on the earth's environment is not favorable. Accordingly, the aforementioned method has problems concerning increase in material costs and complicatedness of disposal of waste liquid.

As a method for reducing the used amount of polyphosphoric acid under a condition of the absence of an organic solvent, there has been proposed a method of using polyphosphoric acid prepared by mixing phosphoric acid and diphosphorus pentoxide so that the resulting mixture has a phosphoric acid concentration of 110 to 130% by weight (Japanese Patent Unexamined Publication No. 2005-289949). Although this method has the advantage of not using an organic solvent, it suffers from inconveniences of preparing polyphosphoric acid just before use, and further, it also suffers from difficulty that it is required to strictly control the amounts of phosphoric acid and diphosphorus pentoxide, and maintain an appropriate temperature of the polyphosphoric acid to keep the viscosity of the polyphosphoric acid being low. If it is taken into consideration that contact of diphosphorus pentoxide with water invites strong heat generation, the temperature control is still more difficult.

As methods for suppressing the amount of polyphosphoric acid used, similarly to the above method, there have been proposed methods of reducing the used amount of polyphosphoric acid to 4 to 6 times of the weight of the starting material by performing the reaction in the presence of an organic solvent such as toluene, xylene, dichloromethane, 1,2-dichloroethane, and benzene (Japanese Patent Unexamined Publication No. 57-171991 (Table 1), Japanese Patent Publication No. 5-41152, Japanese Patent Nos. 2612161 and 2549997).

Furthermore, as a method not using polyphosphoric acid, there has been proposed a method of heat treatment of the compound of the formula (IV) together with phosphoric acid in the presence of an aromatic hydrocarbon type solvent or a halogenated hydrocarbon type solvent (Japanese Patent No. 4603276).

However, when zaltoprofen is produced by the method described in Japanese Patent Publication No. 01-29793 (Example 3) or Japanese Patent Unexamined Publication No. 57-171991 (Table 1), a problem arises that a small amount of impurity presumed to consist of dimmers is produced, of which removal is difficult, and therefore, for supplying the product as a medicament, purity thereof must be improved by repeatedly subjecting the product to a column chromatography treatment or recrystallization (Japanese Patent Unexamined Publication Nos. 2006-273731 and 2005-289949). Similarly as for the method of Japanese Patent No. 4603276, although this patent does not refer to purity of zaltoprofen or generation of impurities, it is considered that the generation of a considerable amount of impurities is unavoidable, because the reaction was performed in dichloroethane at 120°C for 58 hours, in toluene at 120°C for 50 hours, or in chlorobenzene at 150°C for 35 hours according to the method of Japanese Patent No. 4603276, namely a long reaction time as long as 35 to 58 hours.

As a method for solving this problem, there has been proposed a method of suppressing generation of by-products by performing the cyclization reaction in polyphosphoric acid in the presence of an inorganic base such as aluminum hydroxide and/or an inorganic salt such as magnesium chloride (Japanese Patent Unexamined Publication No. 2006-273731). Also as a method for solving the above problem, there have been proposed a method of allowing an equimolar amount of an optically inactive organic amine such as diethylamine to act on a zaltoprofen crude purification product containing a small amount of impurities in an organic solvent, allowing zaltoprofen to crystallize as a crystalline salt with the organic amine (Japanese Patent Publication No. 6-51697), and a method of crystallizing zaltoprofen from a crude purification product thereof containing a small amount of impurities in a ketone solvent such as 2-butanone, acetone, and 4-methyl-2-pentanone (Japanese Patent Unexamined Publication No. 2006-290753).

However, the method of Japanese Patent Unexamined Publication No. 2006-273731 has a problem that the organic solvent used for the cyclization reaction is dichloromethane, which is a substance of which use and disposal should be monitored under the provisions of the "Law Concerning Reporting, etc. of Releases to the Environment of Specific Chemical Substances and Promoting Improvements in Their Management" because of concerns about environmental impact and toxicity to human. Furthermore, the purity of the resulting zaltoprofen was as low as 98.01% (Example 5) to 99.01% (Example 12). Although the purities are higher than the purity of the zaltoprofen obtained without addition of an inorganic base or inorganic salt, i.e., 95.13% (Comparative Example 1) to 96.14% (Comparative Example 2), it is considered that the purities are insufficient for medicaments. In particular, the content of the dimmer, of which removal is extremely difficult, of 0.02% (Example 12) to 0.15% (Example 3) according to the method of Japanese Patent Unexamined Publication No. 2006-273731 is a serious problem when manufacture is carried out in an industrial scale. In addition, the method of Japanese Patent Unexamined Publication No. 2006-273731 also has a problem that when the inorganic base or inorganic salt forms a salt with zaltoprofen, the salt must be converted into the free compound or hydrochloride, which causes a problem of increase of process steps.

Further, the method of Japanese Patent Publication No. 6-51697 requires 3 times of recrystallization, and also has a problem that the crystalline salt with the organic amine must be converted into the free compound or hydrochloride after the recrystallization. Furthermore, in the examples of the method of Japanese Patent Publication No. 6-51697, it is no better than that a spot of impurity was not detected for the purification product obtained by recrystallization when determined by the simple measurement method, i.e., thin layer chromatography, and therefore, it cannot absolutely be understood that no impurities were produced in the method.

The method of Japanese Patent Unexamined Publication No. 2006-290753 requires complicated operations, i.e., after 10 g of the crude purification product is dissolved in 100 ml of a ketone solvent, the solution is evaporated to 20 ml, and returned to room temperature, seed crystals are added, and the mixture is stirred for 12 hours with cooling at 5°C, and then stirred for 1 hour with cooling to -30°C, and the method also has a problem that a large scale equipment is required that enables cooling to -30°C. Furthermore, the method of Japanese Patent Unexamined Publication No. 2006-290753 also has a problem that 0.01% to 0.12% of the dimers still remain unremoved.

### Prior art references

### Patent documents

Patent document 1: Japanese Patent Unexamined Publication No. 55-53282 (Japanese Patent Publication No. 61-7199)
Patent document 2: Japanese Patent Unexamined Publication No. 57-106678 (Japanese Patent Publication No. 01-29793)
Patent document 3: Japanese Patent Unexamined Publication No. 57-171991
Patent document 4: Japanese Patent Unexamined Publication No. 61-251682 (Japanese Patent Publication No. 5-41152)
Patent document 5: Japanese Patent Unexamined Publication No. 62-108877 (Japanese Patent Publication No. 6-51697)
Patent document 6: Japanese Patent Unexamined Publication No. 62-292780 (Japanese Patent No. 2612161)
Patent document 7: Japanese Patent Unexamined Publication No. 8-99953 (Japanese Patent No. No. 2549997)
Patent document 8: Japanese Patent Unexamined Publication No. 2005-247778 (Japanese Patent No. 4603276)
Patent document 9: Japanese Patent Unexamined Publication No. 2005-289949
Patent document 10: Japanese Patent Unexamined Publication No. 2006-273731
Patent document 11: Japanese Patent Unexamined Publication No. 2006-290753

### Summary of the Invention

### Object to be Achieved by the Invention

As described above, there have been proposed methods of cyclizing only one of the carboxylic acid groups of the compound of the formula (IV) by a Friedel-Crafts reaction type intramolecular cyclization reaction in the presence of an organic solvent.

However, in the method of cyclizing a compound of the formula (B) or the formula (IV) by a Friedel-Crafts reaction type intramolecular cyclization reaction, there is not known any method for preparing zaltoprofen or a zaltoprofen derivative in an industrial scale with a purity sufficient for use as an active ingredient of medicaments at an industrially acceptable yield in a simple and economical manner, without generating a detectable amount of impurities such as the dimmers, using polyphosphoric acid in an amount of 6.5 times or less of the amount of the compound of the formula (B) or formula (IV) as a condensing agent.

Therefore, there is desired a novel method for preparing zaltoprofen or a zaltoprofen derivative in an industrial scale with a purity sufficient for use as an active ingredient of medicaments at an industrially acceptable yield in a simple and economical manner, without generating a detectable amount of impurities such as the dimmers, by cyclizing a compound of the formula (II) or the formula (IV) by a Friedel-Crafts reaction type intramolecular cyclization reaction using polyphosphoric acid in an amount of 6.5 times or less of the amount of the compound of the formula (II) or formula (IV) as a condensing agent.

The solvent used in the method is required to enable the production of zaltoprofen or a zaltoprofen derivative with a high purity at a sufficiently high reaction rate, and simple and convenient isolation and purification of the product, and for that purpose, it is also required to prepare zaltoprofen or a zaltoprofen derivative by using a solvent having a boiling point of 80°C or higher and hardly soluble or insoluble in water, which is, in addition, not one of organic solvents defined in the "Impurities: Guideline for Residual Solvents" as those "use should be avoided in manufacture of pharmaceutical additives and preparations".

As organic solvents usable in the method of cyclizing a compound of the formula (II) or the formula (IV) by a Friedel-Crafts reaction type intramolecular cyclization reaction, Japanese Patent Publication No. 61-7199 refers to benzene, toluene, and xylene, Japanese Patent Unexamined Publication No. 57-171991 refers to dichloromethane, 1,2-dichloroethane, toluene, xylene, and benzene, Japanese Patent Publication No. 5-41152 refers to dichloromethane, toluene, and benzene, Japanese Patent Publication No. 6-51697 refers to dichloromethane, 1,2-dichloroethane, chloroform, benzene, toluene, and xylene, Japanese Patent Nos. 2612161 and 2549997 mention dichloromethane, Japanese Patent No. 4603276 refers to benzene, toluene, dichloromethane, dichloroethane, trichloroethane, chlorobenzene, and dichlorobenzene, and Japanese Patent Unexamined Publication Nos. 2005-289949, 2006-273731, and 2006-290753 refer to dichloromethane, chloroform, carbon tetrachloride, toluene, chlorobenzene, nitrobenzene, hexane, heptane, diethyl ether, diisopropyl ether, and dimethyl carbonate.

Although a method of cyclizing the compound of the formula (IV) in the presence of heptane, among these organic solvents, is exemplified in Japanese Patent Unexamined Publication No. 2006-273731, the advantage effect thereof is not verified by the example, and the patent document fails to specifically explain that n-heptane can be used among various heptanes.

Further, among the aforementioned organic solvents, those of which advantageous effect has been specifically verified are only 1,2-dichloroethane (Japanese Patent Unexamined Publication No. 57-171991, Japanese Patent No. 4603276), dichloromethane (Japanese Patent Unexamined Publication No. 57-171991, Japanese Patent Publication No. 5-41152, Japanese Patent Nos. 2612161, 2549997, and Japanese Patent Unexamined Publication No. 2005-289949), toluene (Japanese Patent Unexamined Publication No. 57-171991, Japanese Patent No. 4603276), xylene (Japanese Patent Unexamined Publication No. 57-171991), and chlorobenzene (Japanese Patent No. 4603276).

In addition, each of these organic solvents has a significant problem when used in industrial production of a medicament.

For example, although dichloromethane is fire resistant, it has a risk of explosion, and generates harmful phosgene, hydrogen chloride, and chlorine when it is exposed to fire. Moreover, the solvent is a class 2 organic solvent defined to be a substance of which "residual amount should be regulated in order to protect a patient from possible harmful actions thereof", and is also a substance of which use and disposal should be monitored under the provisions of the "Law Concerning Reporting, etc. of Releases to the Environment of Specific Chemical Substances and Promoting Improvements in Their Management" because of the concerns about environmental impact and toxicity against human. Furthermore, since the solvent causes vertigo, lethargy, headache, nausea, feeling of weakness, and unconsciousness, it is desirable to avoid use thereof in manufacture of medicaments.

Toluene is a class 2 organic solvent, and volatile at ordinary temperature, and has inflammability, and since a gaseous mixture of vapor thereof and air has explosiveness, it imposes high risk of fire. Moreover, since inhalation of toluene gives potent anesthetic action or hypnotic action, and causes cough, pharyngalgia, vertigo, lethargy, headache, nausea, and unconsciousness, it is desirable to avoid use thereof in manufacture of medicaments. Toluene is an organic solvent hardly soluble in water, and the boiling point of toluene is 111°C.

Each of o-xylene, m-xylene, and p-xylene has inflammability, and may produce an explosive gaseous mixture of vapor thereof and air at a temperature of 32°C or higher, and accordingly, the solvent imposes high risk of fire. Moreover, xylene is a class 2 organic solvent, and is also a substance of which use and disposal should be monitored under the Poisonous and Deleterious Substances Control Law and the "Law Concerning Reporting, etc. of Releases to the Environment of Specific Chemical Substances and Promoting Improvements in Their Management". Further, inhalation thereof provides potent anesthetic action or hypnotic action, and also causes vertigo, lethargy, headache, and nausea, and therefore it is desirable to avoid use thereof in manufacture of medicaments. Each of o-xylene, m-xylene, and p-xylene is an organic solvent hardly soluble in water, and the boiling points of o-xylene, m-xylene, and p-xylene are 144°C, 139°C, and 138°C, respectively.

1,2-Dichloroethane has high inflammability, a gaseous mixture of vapor thereof and air shows explosiveness, and since the solvent contains chlorine, it imposes concerns about environmental pollution and concerns about generation of harmful hydrogen chloride and phosgene. Moreover, since 1,2-dichloroethane is harmful to human body and environment, it is classified to be a class 1 organic solvent defined in the "Impurities: Guideline for Residual Solvents" notified by the Ministry of Health and Welfare in 1998 that "use thereof should be avoided in the production of drug substances, excipients, or drug products unless use thereof can be strongly justified in a risk-benefit assessment". Further, since inhalation thereof by factory workers causes abdominal pain, cough, vertigo, lethargy, headache, nausea, pharyngalgia, unconsciousness, and vomiting, it is desirable to avoid the use thereof in manufacture of medicaments. 1,2-Dichloroethane is an organic solvent hardly soluble in water, and the boiling point thereof is 83.5°C.

Chlorobenzene is a class 2 organic solvent, and has inflammability, and the solvent may produce an explosive gaseous mixture of vapor thereof and air at a temperature of 27°C or higher. Moreover, since the solvent contains chlorine, it imposes concerns about environmental pollution and concerns about generation of harmful hydrogen chloride and phosgene. Furthermore, inhalation of chlorobenzene causes inhalation lethargy, headache, nausea, and unconsciousness, it is desirable to avoid use thereof in manufacture of medicaments. Chlorobenzene is an organic solvent hardly soluble in water, and the boiling point of chlorobenzene is 132°C.

Chloroform is a class 2 organic solvent, and since it contains chlorine, it imposes concerns about environmental pollution and concerns about generation of harmful hydrogen chloride and phosgene. Therefore, it is desirable to avoid use thereof in manufacture of medicaments. Furthermore, inhalation of chloroform causes inhalation lethargy, headache, nausea, and unconsciousness, and provides potent anesthetic action or hypnotic action. Chloroform is an organic solvent that dissolves in water up to 0.82%, and the boiling point of chloroform is 61°C.

As described above, dichloromethane, toluene, xylene, 1,2-dichloroethane, chlorobenzene, and chloroform are organic solvents of which use is desirably avoided in manufacture of medicaments.

### Means for Achieving the Object

Under the aforementioned situation, the inventor of the present invention recognized, for the first time, a novel object of finding an organic solvent as an optimum organic solvent used in the method for preparing zaltoprofen or a zaltoprofen derivative by cyclizing a compound of the formula (II) or (IV) by a Friedel-Crafts reaction type intramolecular cyclization reaction, and examined various organic solvents in a trial and error manner. As a result, the inventor of the present invention found that one or more kinds of organic solvents selected from the group consisting of 1,1,2-trichloroethene, methylcyclohexane, cyclohexane, n-heptane, acetonitrile, 1,2-dimethoxyethane, 1,4-dioxane, tetrahydrofuran, N,N-dimethylformamide, ethyl acetate, and methyl ethyl ketone are preferred, and one or more kinds of organic solvents selected from the group consisting of 1,1,2-trichloroethene, methylcyclohexane, cyclohexane, and n-heptane are especially preferred, and accomplished the present invention.

The present invention thus relates to a method for synthesizing a compound of the formula (I) or (III), which comprises cyclizing a compound of the formula (II) or (IV) by a Friedel-Crafts reaction type intramolecular cyclization reaction in the presence of one or more kinds of organic solvents selected from the group consisting of 1,1,2-trichloroethene, methylcyclohexane, cyclohexane, n-heptane, acetonitrile, 1,2-dimethoxyethane, 1,4-dioxane, tetrahydrofuran, N,N-dimethylformamide, ethyl acetate, and methyl ethyl ketone.

Furthermore, the temperature suitable for the heat treatment for performing the cyclization reaction remained unknown. The inventor of the present invention found that when these organic solvents were used, a temperature near the boiling point of the organic solvent used was preferred, and thus the characteristic feature of the present invention was made more definite. Specifically, the temperature suitable for the heat treatment for performing the cyclization reaction is in the range of from minus 25°C to plus 25°C relative to the boiling point of the organic solvent used as the reference temperature, preferably a temperature in the range of from minus 20°C to plus 20°C relative to the boiling point of the organic solvent used, more preferably a temperature in the range of from minus 15°C to plus 15°C relative to the boiling point of the organic solvent used, still more preferably a temperature in the range of from minus 10°C to plus 10°C relative to the boiling point of the organic solvent used, and most preferably a temperature in the range of from minus 5°C to plus 5°C relative to the boiling point of the organic solvent used.

Specifically, the present inventions are defined as follows.

[1] A method for preparing a compound represented by the following formula (I): 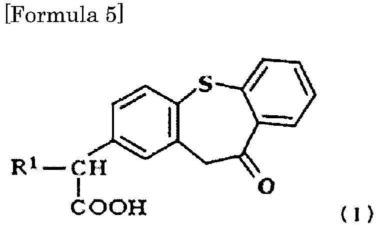 wherein, in the formula (I);
   R¹ is H or a hydrocarbon group having 1 to 8 carbon atoms, which comprises cyclizing a compound represented by the following formula (II): 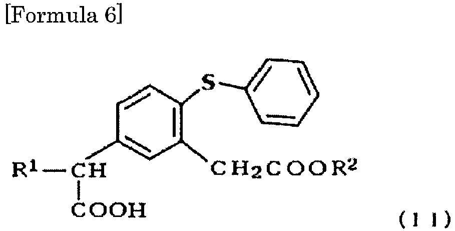
   wherein, in the formula (II),
   R¹ is H or a hydrocarbon group having 1 to 8 carbon atoms; and
   R² is H or a hydrocarbon group having 1 to 8 carbon atoms;
   by subjecting the compound of the formula (II) to a heat treatment together with at least one kind of condensing agent selected from the group consisting of polyphosphoric acid, orthophosphoric acid, and concentrated sulfuric acid,
   in the presence of at least one kind of organic solvent selected from the group consisting of 1,1,2-trichloroethene, methylcyclohexane, cyclohexane, n-heptane, acetonitrile, 1,2-dimethoxyethane, 1,4-dioxane, tetrahydrofuran, N,N-dimethylformamide, ethyl acetate, and methyl ethyl ketone.
[2] The method for preparation according to [1], wherein the organic solvent is at least one kind of organic solvent selected from the group consisting of 1,1,2-trichloroethene, methylcyclohexane, cyclohexane, and n-heptane.
[3] The method for preparation according to [1] or [2], wherein the condensing agent is polyphosphoric acid.
[4] The method for preparation according to any one of [1] to [3], wherein the heat treatment is performed at a temperature in the range of from minus 25°C to plus 25°C relative to the boiling point of the organic solvent used as the reference temperature.
[5] The method for preparation according to any one of [1] to [4], wherein time of the heat treatment is 1 to 8 hours.
[6] The method for preparation according to any one of [1] to [5], wherein weight of the condensing agent is preferably 1 to 10 times, more preferably 1 to 6.5 times, of the weight of the compound represented by the formula (II).
[7] The method for preparation according to any one of [1] to [6], wherein volume of the organic solvent is 50 to 100 mL per 50 g of the compound represented by the formula (II).
[8] The method for preparation according to any one of [1] to [7], wherein R¹ is methyl group.
[9] The method for preparation according to any one of [1] to [8], wherein R² is H or methyl group.
[10] The method for preparation according to any one of [1] to [9], wherein polyphosphoric acid having a condensation ratio of 105% or higher is used.
[11] The method for preparation according to any one of [1] to [10], which further comprises extracting zaltoprofen or zaltoprofen derivative by using ethyl acetate.
[12] The method for preparation according to any one of [1] to [11], which further comprises drying zaltoprofen or zaltoprofen derivative over anhydrous sodium sulfate.
[13] The method for preparation according to any one of [1] to [12], which further comprises adding an organic solvent to zaltoprofen or zaltoprofen derivative, dissolving zaltoprofen or zaltoprofen derivative by warming, and removing insoluble matter by filtration.
[14] The method for preparation according to any one of [1] to [13], which further comprises adding an organic solvent to zaltoprofen or zaltoprofen derivative, dissolving zaltoprofen or zaltoprofen derivative by warming, and then crystallizing zaltoprofen or zaltoprofen derivative by lowering the temperature.

### Effect of the Invention

According to the method for preparation of the present invention, the compound of the formula (II) or (IV) is cyclized by a Friedel-Crafts reaction type intramolecular cyclization reaction in the presence of one or more kinds of organic solvents selected from the group consisting of 1,1,2-trichloroethene, methylcyclohexane, cyclohexane, n-heptane, acetonitrile, 1,2-dimethoxyethane, 1,4-dioxane, tetrahydrofuran, N,N-dimethylformamide, ethyl acetate, and methyl ethyl ketone, and as a result, zaltoprofen or a zaltoprofen derivative can be prepared at a purity of 99% or higher and a yield of 60% or higher by using polyphosphoric acid in an amount of 6.5 times or less of the amount of the compound of the formula (II) or (IV) as a condensing agent.

When at least one kind of organic solvent selected from the group consisting of methylcyclohexane, cyclohexane, and n-heptane is used as a more preferred organic solvent in the method for preparation of the present invention, factory workers engaged in manufacture are free from concerns about potent anesthetic action or hypnotic action, even if they inhale vapor of the organic solvent, and since they are organic solvents not containing chlorine, there is no concern about environmental pollution, and no concern about generation of harmful hydrogen chloride and phosgene even in an emergency.

Since an organic solvent having a boiling point of 80°C or higher is used in the method for preparation of the present invention, the Friedel-Crafts reaction type intramolecular cyclization reaction can be performed at a high temperature, and therefore zaltoprofen or a zaltoprofen derivative of a purity of 99% or higher can be prepared at a yield of 60% or higher with a reaction time of 8 hours or shorter. In particular, when at least one kind of organic solvent selected from the group consisting of methylcyclohexane, cyclohexane, n-heptane, and 1,1,2-trichloroethene is used as the solvent used for the cyclization reaction, zaltoprofen or a zaltoprofen derivative of a purity of 99.5% or higher, or 99.8% or higher can be prepared.

According to the method for preparation of the present invention, a hardly water-soluble or water-insoluble organic solvent is used, and therefore separation and purification of produced zaltoprofen or a zaltoprofen derivative are readily achievable.

According to the method for preparation of the present invention, the organic solvent used for the reaction can be obtained at a sufficiently economical price.

According to the method for preparation of the present invention, use of class 1 organic solvents defined in the "Impurities: Guideline for Residual Solvents" that "use thereof should be avoided in the production of drug substances, excipients, or drug products unless use thereof can be strongly justified in a risk-benefit assessment" can be avoided.

According to the method for preparation of the present invention, with a small number of preparation steps, zaltoprofen or a zaltoprofen derivative having a purity of 99% or higher can be prepared, and generation of the dimer is not detected.

According to the method for preparation of the present invention, zaltoprofen or a zaltoprofen derivative can be prepared in an industrial scale in a simple and economical manner.

Moreover, according to the method for preparation of the present invention, the number of preparation steps can be reduced.

Furthermore, according to the method for preparation of the present invention, workers on manufacturing sites hardly suffer from concerns about health hazard, and can produce zaltoprofen or a zaltoprofen derivative by using an organic solvent that can be easily handled in the production operations.

### Brief Description of the Drawings

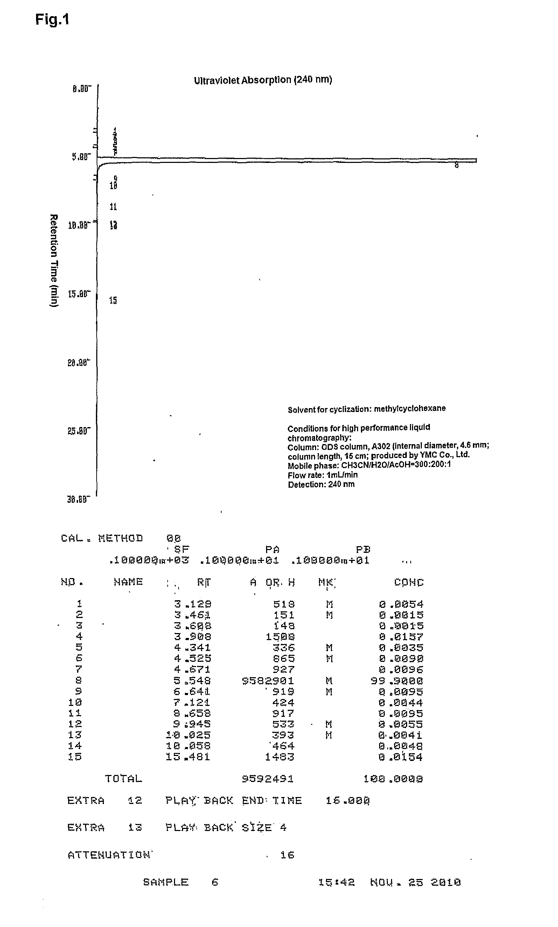

[Fig. 1] Fig. 1 shows a high performance liquid chromatography chart of zaltoprofen prepared by cyclization performed in the presence of methylcyclohexane. The numbers indicated in the longitudinal direction represent retention time (RT) in terms of minute. The peak in the transverse direction represents ultra violet absorbance at a wavelength of 240 nm. In the table shown under the high performance liquid chromatography chart, NO. represents peak number, RT represents retention time, "A OR H" represents peak area, and CONC represents ratio to the whole peak area (Example 1). As far as observation is made on the chart, any peaks other than the peak of zaltoprofen are not seen. As for the conditions of the high performance liquid chromatography, an ODS column produced by YMC Co., Ltd., A302 (internal diameter, 4.6 mm; column length, 15 cm) was used, the mobile phase was CH₃CN/H₂O/AcOH = 300:200:1, the flow rate was 1 mL/min, and the detection was based on ultra violet absorbance at a wavelength of 240 nm. In the figure, the unit of the retention time (R.T.) mentioned in the HPLC chart is minute.

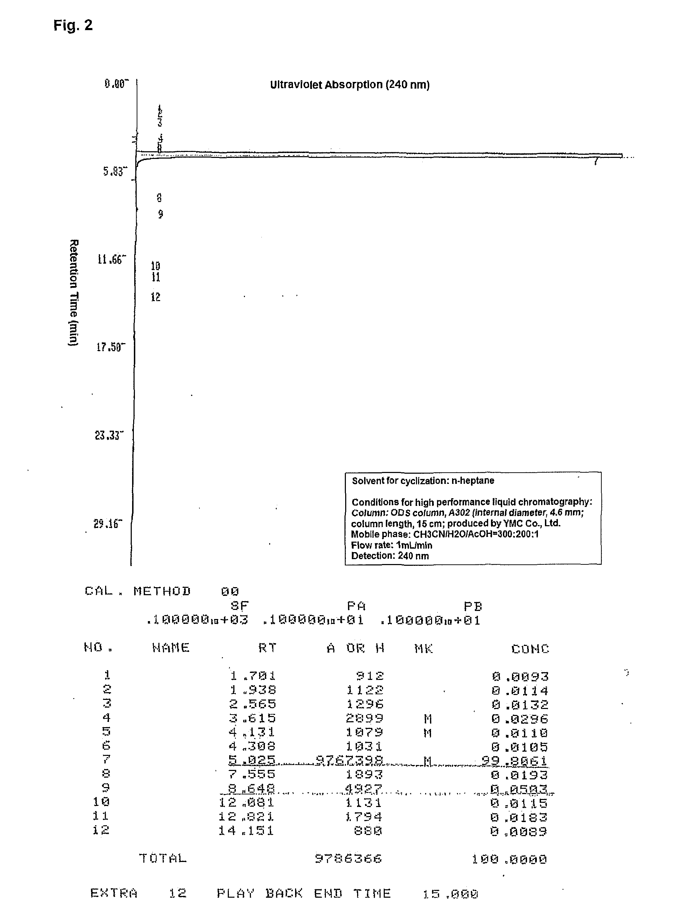

[Fig. 2] Fig. 2 shows a high performance liquid chromatography chart of zaltoprofen prepared by cyclization performed in the presence of n-heptane (Example 2). The conditions of the high performance liquid chromatography, and meanings of the indications used in the figure are the same as those of Fig. 1. As far as observation is made on the chart, any peaks other than the peak of zaltoprofen are not seen.

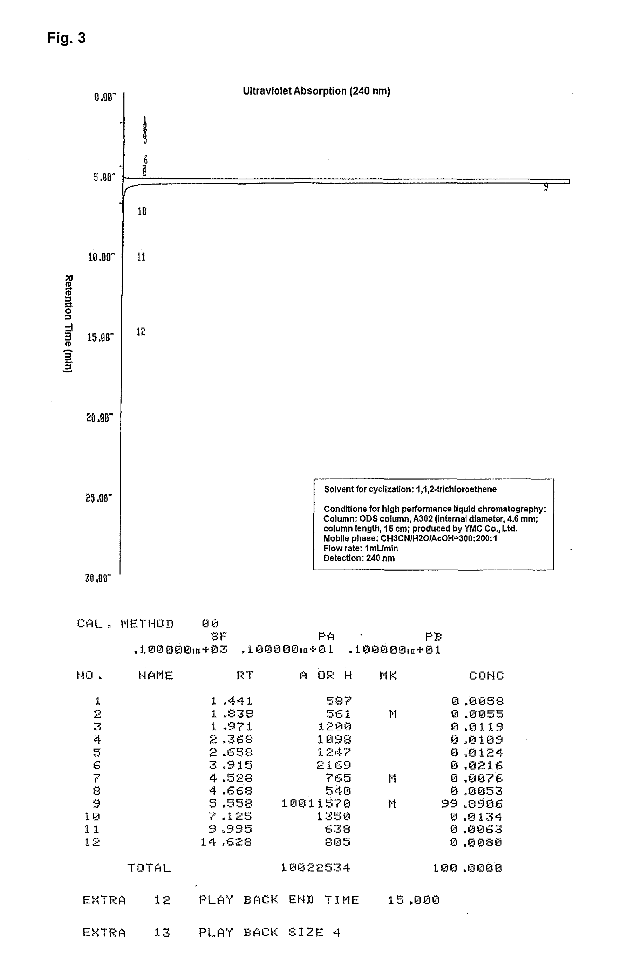

[Fig. 3] Fig. 3 shows a high performance liquid chromatography chart of zaltoprofen prepared by cyclization performed in the presence of 1,1,2-trichloroethene (Example 3). The conditions of the high performance liquid chromatography, and meanings of the indications used in the figure are the same as those of Fig. 1. As far as observation is made on the chart, any peaks other than the peak of zaltoprofen are not seen.

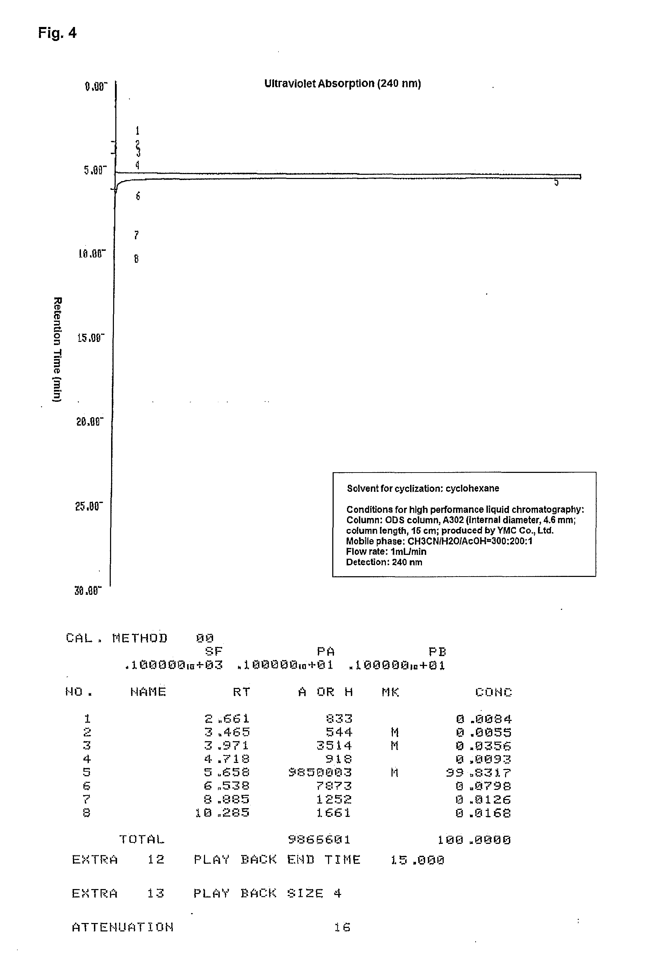

[Fig. 4] Fig. 4 shows a high performance liquid chromatography chart of zaltoprofen prepared by cyclization performed in the presence of cyclohexane (Example 4). The conditions of the high performance liquid chromatography, and meanings of the indications used in the figure are the same as those of Fig. 1. As far as observation is made on the chart, any peaks other than the peak of zaltoprofen are not seen.

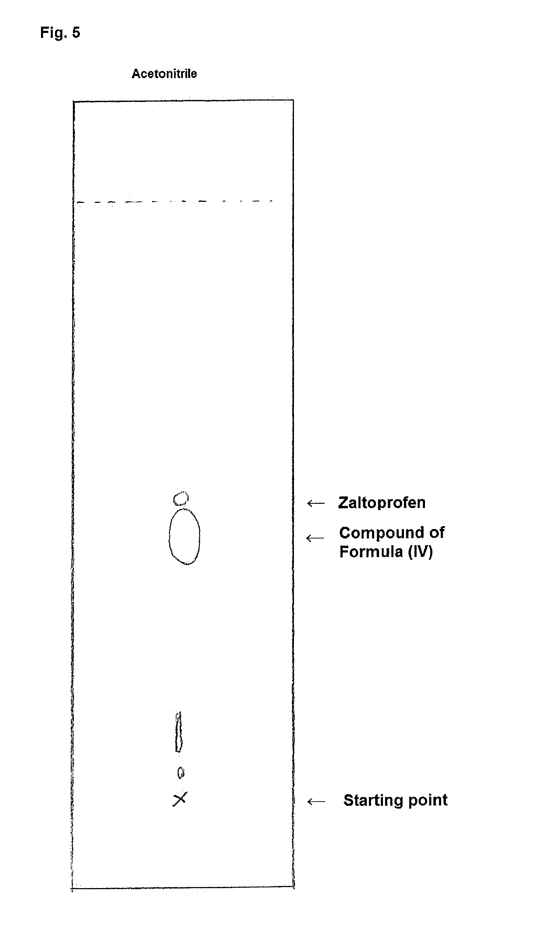

[Fig. 5] Fig. 5 shows a sketch of a thin layer chromatography developed image of a product of the cyclization reaction performed in the presence of acetonitrile (Example 5). The thin layer chromatography was carried out by spotting a sample of the reaction product on Silica Gel 60F254 produced by Merck, developing the spot with a solvent of CHCl₃:MeOH:AcOH = 100:10:1, and detecting the developed spot on the basis of ultraviolet absorption at a wavelength of 254 nm. The broken line shown in the figure indicates the position of the solvent front. Although production of zaltoprofen was observed, there were also observed unreacted compound of the formula (IV), and a small amount of impurity as a spot near the starting point.

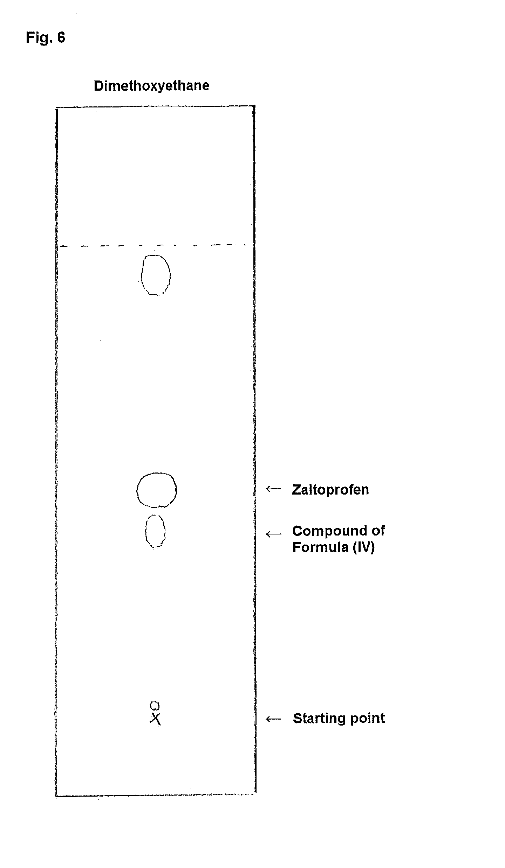

[Fig. 6] Fig. 6 shows a sketch of a thin layer chromatography developed image of a product of the cyclization reaction performed in the presence of dimethoxyethane (Example 6). The conditions of the thin layer chromatography, and meanings of the indications used in the figure are the same as those of Fig. 5. Although production of zaltoprofen was observed as a major spot, there were also observed unreacted compound of the formula (IV), a small amount of impurity (spot near the starting point), and unknown impurity (spot near the solvent front).

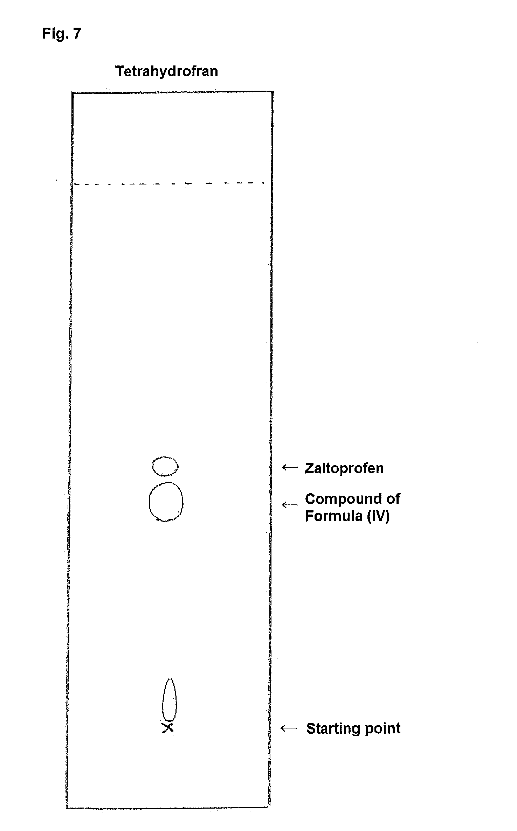

[Fig. 7] Fig. 7 shows a sketch of a thin layer chromatography developed image of a product of the cyclization reaction performed in the presence of tetrahydrofuran (Example 8). The conditions of the thin layer chromatography, and meanings of the indications used in the figure are the same as those of Fig. 5. Production of zaltoprofen was clearly observed, and there were also observed unreacted compound of the formula (IV), and a small amount of impurity (spot near the starting point).

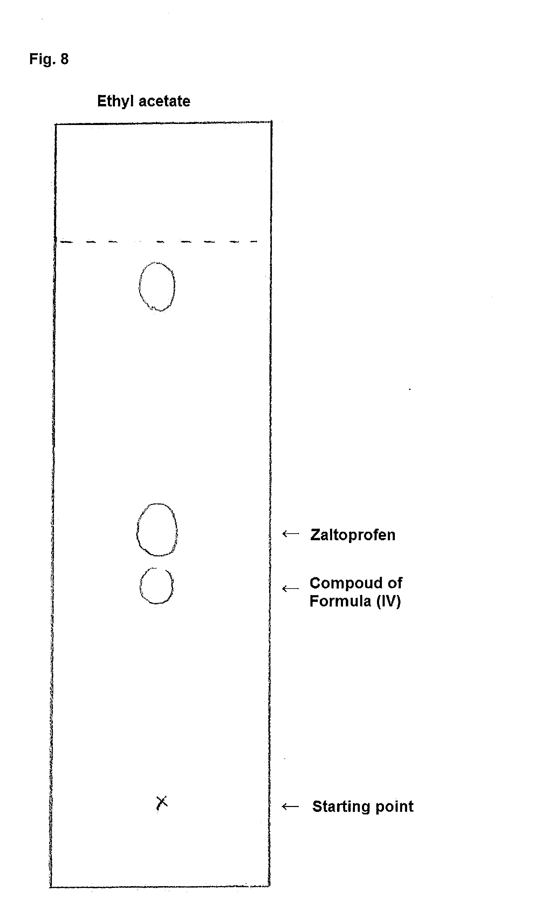

[Fig. 8] Fig. 8 shows a sketch of a thin layer chromatography developed image of a product of the cyclization reaction performed in the presence of ethyl acetate (Example 10). The conditions of the thin layer chromatography, and meanings of the indications used in the figure are the same as those of Fig. 5. It was observed that the major product was zaltoprofen, and there were also observed unreacted compound of the formula (IV), and an impurity considered to be a decomposition product (spot near the solvent front).

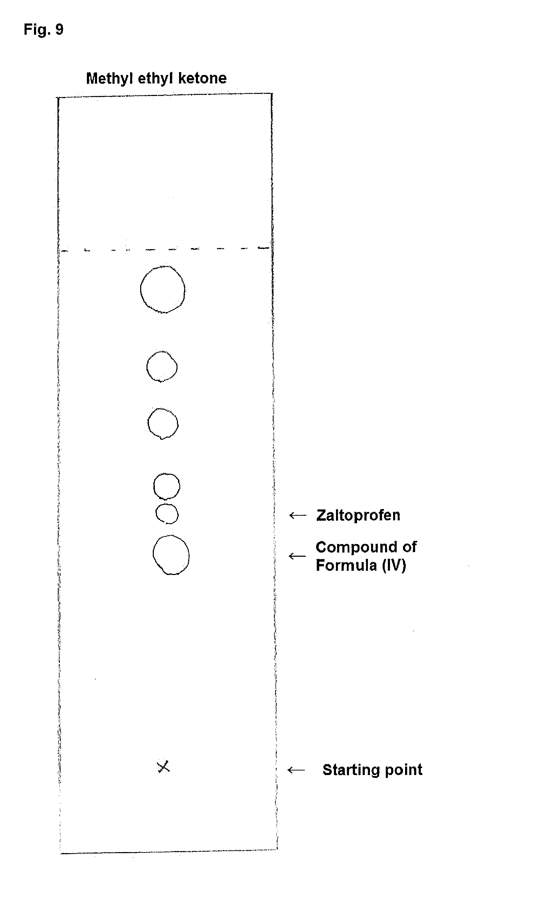

[Fig. 9] Fig. 9 shows a sketch of a thin layer chromatography developed image of a product of the cyclization reaction performed in the presence of methyl ethyl ketone (Example 11). The conditions of the thin layer chromatography, and meanings of the indications used in the figure are the same as those of Fig. 5. Production of zaltoprofen was observed, and there were also observed unreacted compound of the formula (IV), and four kinds of impurities considered to be decomposition products (spots between the spot of zaltoprofen and the solvent front).

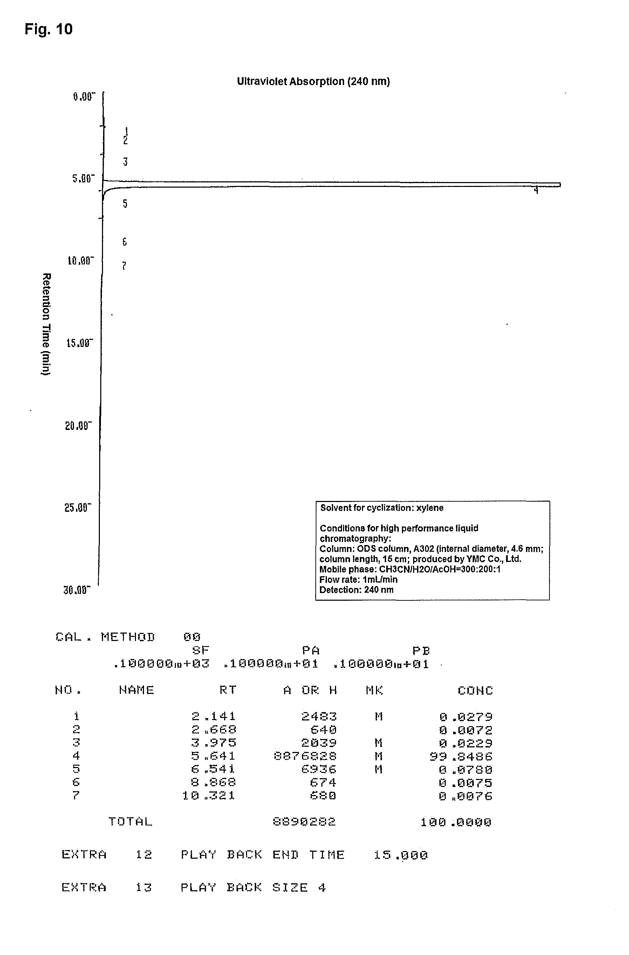

[Fig. 10] Fig. 10 shows a high performance liquid chromatography chart of zaltoprofen prepared by cyclization performed in the presence of xylene (Comparative Example 1). The conditions of the high performance liquid chromatography, and meanings of the indications used in the figure are the same as those of Fig. 1. As far as observation is made on the chart, any peaks other than the peak of zaltoprofen are not seen.

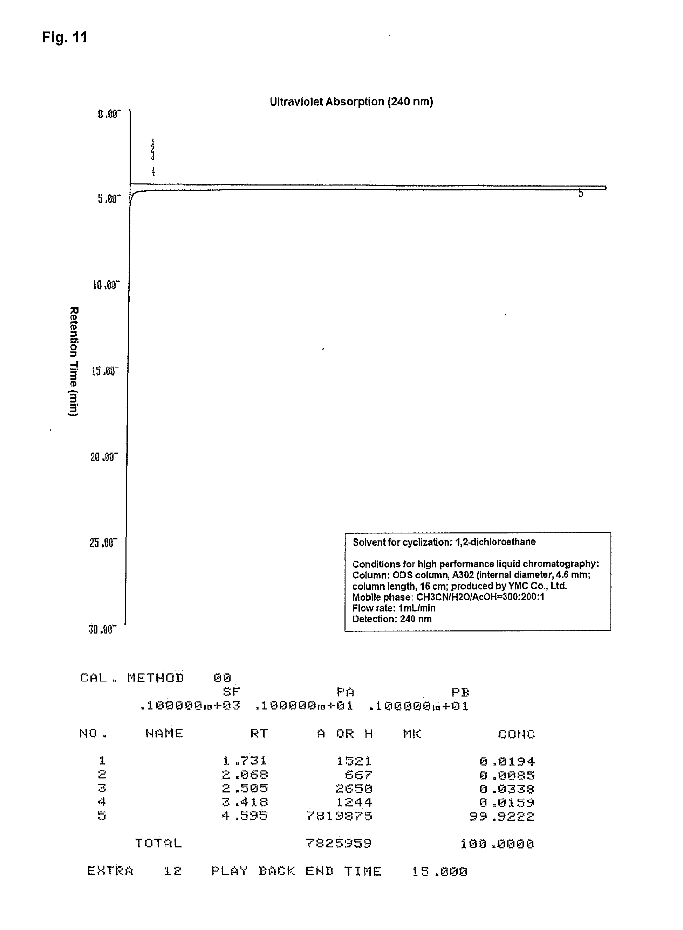

[Fig. 11] Fig. 11 shows a high performance liquid chromatography chart of zaltoprofen prepared by cyclization performed in the presence of 1,2-dichloroethane (Comparative Example 2). The conditions of the high performance liquid chromatography, and meanings of the indications used in the figure are the same as those of Fig. 1. As far as observation is made on the chart, any peaks other than the peak of zaltoprofen are not seen.

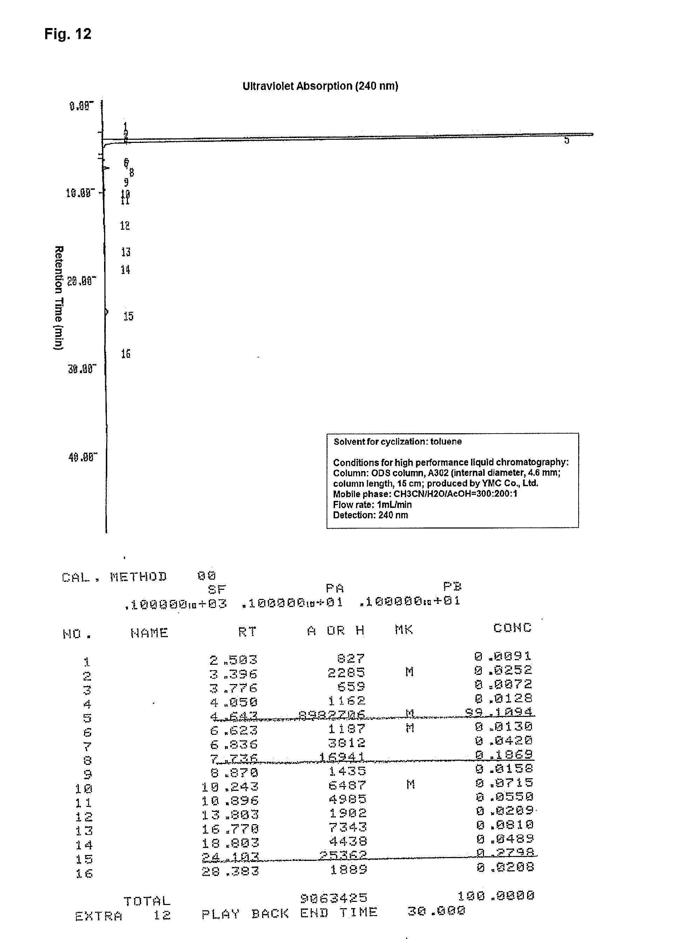

[Fig. 12] Fig. 12 shows a high performance liquid chromatography chart of zaltoprofen prepared by cyclization performed in the presence of toluene (Comparative Example 3). The conditions of the high performance liquid chromatography, and meanings of the indications used in the figure are the same as those of Fig. 1. Other than the peak of zaltoprofen, several small peaks can be observed.

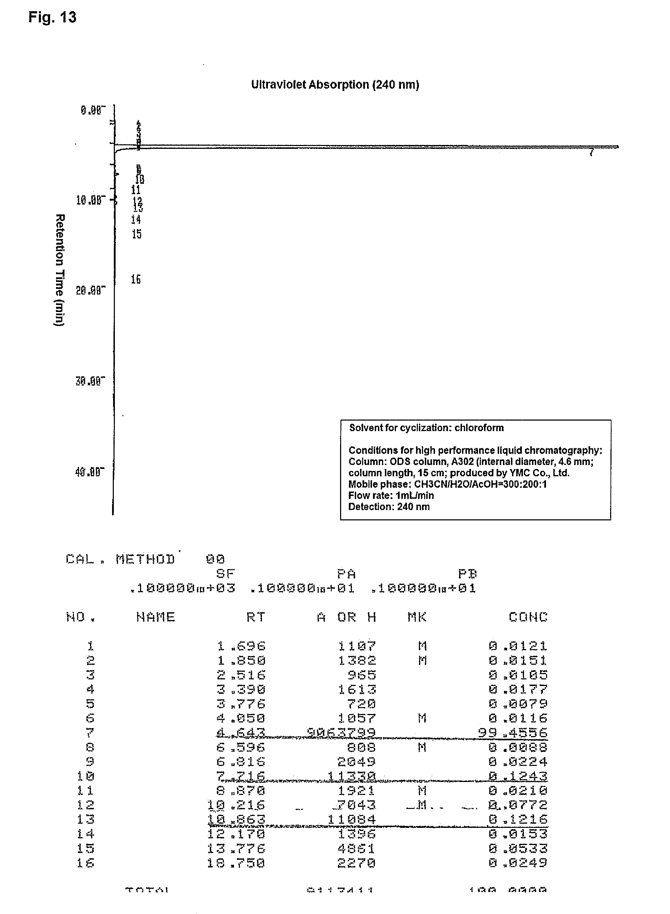

[Fig. 13] Fig. 13 shows a high performance liquid chromatography chart of zaltoprofen prepared by cyclization performed in the presence of chloroform (Comparative Example 4). The conditions of the high performance liquid chromatography, and meanings of the indications used in the figure are the same as those of Fig. 1. Other than the peak of zaltoprofen, several small peaks can be observed.

### Modes for Carrying out the Invention

### Organic solvent

The organic solvent used in the present invention consists of one or more kinds of organic solvents selected from the group consisting of methylcyclohexane, cyclohexane, n-heptane, 1,1,2-trichloroethene, acetonitrile, dimethoxyethane, 1,4-dioxane, tetrahydrofuran, N,N-dimethylformamide, ethyl acetate, and methyl ethyl ketone. A preferred organic solvent used in the present invention consists of one or more kinds of organic solvents selected from the group consisting of methylcyclohexane, cyclohexane, n-heptane and 1,1,2-trichloroethene, a more preferred organic solvent used in the present invention consists of one or more kinds of organic solvents selected from the group consisting of methylcyclohexane, cyclohexane, and n-heptane, and a still more preferred organic solvent used in the present invention consists of one or more kinds of organic solvents selected from the group consisting of methylcyclohexane, and n-heptane.

Among the organic solvents used in the method for preparation of the present invention, for example, methylcyclohexane is a water-insoluble organic solvent, and the boiling point thereof is 101°C. Since methylcyclohexane is an organic solvent, it has slight anesthetic action, but it does not have strong anesthetic action. Although methylcyclohexane is classified as a class 2 organic solvent defined to be a substance of which "residual amount should be regulated in order to protect patient from possible harmful actions thereof' in "Impurities: Guideline for Residual Solvents", it is not an organic solvent of which use should be avoided.

Among the organic solvents used in the method for preparation of the present invention, n-heptane is a water-insoluble organic solvent, and the boiling point thereof is 98.4°C. Since n-heptane is an organic solvent, it has slight anesthetic action, but it does not have strong anesthetic action. n-Heptane is classified as a class 3 organic solvent defined to be a "solvent of low toxicity" "that should be used" in "Impurities: Guideline for Residual Solvents".

Among the organic solvents used in the method for preparation of the present invention, cyclohexane is a water-insoluble organic solvent, and the boiling point thereof is 81.4°C. Since cyclohexane is an organic solvent, it has slight anesthetic action, but it does not have strong anesthetic action. Although cyclohexane is classified as a class 2 organic solvent defined to be a substance of which "residual amount should be regulated in order to protect patient from possible harmful actions thereof" in "Impurities: Guideline for Residual Solvents", it is not an organic solvent of which use should be avoided.

Among the organic solvents used in the method for preparation of the present invention, 1,1,2-trichloroethene is a hardly water-soluble organic solvent, and the boiling point thereof is 87.2°C. 1,1,2-Trichloroethene has strong anesthetic action, and since it is an organic solvent containing chlorine, it imposes concerns about environmental pollution, and concerns about generation of harmful hydrogen chloride and phosgene. Although 1,1,2-trichloroethene is classified as a class 2 organic solvent defined to be a substance of which "residual amount should be regulated in order to protect patient from possible harmful actions thereof" in "Impurities: Guideline for Residual Solvents", it is not an organic solvent of which use should be avoided.

Among the organic solvents used in the method for preparation of the present invention, acetonitrile is an organic solvent extremely easily dissolvable in water, and the boiling point thereof is 82°C. Since acetonitrile is an organic solvent, it has slight anesthetic action, but it does not have strong anesthetic action. Although acetonitrile is classified as a class 2 organic solvent defined to be a substance of which "residual amount should be regulated in order to protect patient from possible harmful actions thereof' in "Impurities: Guideline for Residual Solvents", it is not an organic solvent of which use should be avoided.

Among the organic solvents used in the method for preparation of the present invention, 1,2-dimethoxyethane is an organic solvent extremely easily dissolvable in water, and the boiling point thereof is 82°C. Since 1,2-dimethoxyethane is an organic solvent, it has slight anesthetic action, but it does not have strong anesthetic action. Although 1,2-dimethoxyethane is classified as a class 2 organic solvent defined to be a substance of which "residual amount should be regulated in order to protect patient from possible harmful actions thereof" in "Impurities: Guideline for Residual Solvents", it is not an organic solvent of which use should be avoided.

Among the organic solvents used in the method for preparation of the present invention, 1,4-dioxane is a water-miscible organic solvent, and the boiling point thereof is 101°C. Since 1,4-dioxane is an organic solvent, it has slight anesthetic action, but it does not have strong anesthetic action. Although 1,4-dioxane is classified as a class 2 organic solvent defined to be a substance of which "residual amount should be regulated in order to protect patient from possible harmful actions thereof" in "Impurities: Guideline for Residual Solvents", it is not an organic solvent of which use should be avoided.

Among the organic solvents used in the method for preparation of the present invention, tetrahydrofuran is a water-miscible organic solvent, and the boiling point thereof is 66°C. Since tetrahydrofuran is an organic solvent, it has slight anesthetic action, but it does not have strong anesthetic action. Although tetrahydrofuran is classified as a class 2 organic solvent defined to be a substance of which "residual amount should be regulated in order to protect patient from possible harmful actions thereof' in "Impurities: Guideline for Residual Solvents", it is not an organic solvent of which use should be avoided.

Among the organic solvents used in the method for preparation of the present invention, N,N-dimethylformamide is a water-miscible organic solvent, and the boiling point thereof is 153°C. Since N,N-dimethylformamide is an organic solvent, it has weak anesthetic action, but it does not have strong anesthetic action. Although N,N-dimethylformamide is classified as a class 2 organic solvent defined to be a substance of which "residual amount should be regulated in order to protect patient from possible harmful actions thereof" in "Impurities: Guideline for Residual Solvents", it is not an organic solvent of which use should be avoided.

Among the organic solvents used in the method for preparation of the present invention, ethyl acetate is an organic solvent that is dissolved in water up to 8.3 g/100 mL, and the boiling point thereof is 77.2°C. Since ethyl acetate is an organic solvent, it has weak anesthetic action, but it does not have strong anesthetic action. Ethyl acetate is classified as a class 3 organic solvent defined to be a "solvent of low toxicity" "that should be used" in "Impurities: Guideline for Residual Solvents".

Among the organic solvents used in the method for preparation of the present invention, methyl ethyl ketone is an organic solvent that is dissolved in water up to 29 g/100 mL, and the boiling point thereof is 79.6°C. Since methyl ethyl ketone is an organic solvent, it has weak anesthetic action, but it does not have strong anesthetic action. Methyl ethyl ketone is classified as a class 3 organic solvent defined to be a "solvent of low toxicity" "that should be used" in "Impurities: Guideline for Residual Solvents".

### Condensing agent

Condensing agents usable in the present invention are polyphosphoric acid, phlyphosphoric acid ester, phosphoric acid, sulfuric acid, aluminum chloride, boron trifluoride, and tin chloride. The condensing agent used in the present invention is preferably polyphosphoric acid.

Polyphosphoric acid used in the present invention is a linear polymer phosphoric acid produced by dehydration condensation of orthophosphoric acid. Metaphosphoric acid represented by the formula (HPO₃)ₙ also falls within the scope of polyphosphoric acid. Polyphosphoric acid is represented by the general formula Hₙ₊₂PₙO₃ₙ₊₁ (n is an integer of 2 or larger). Examples of polyphosphoric acid include diphosphoric acid, triphosphoric acid, tetraphosphoric acid, pentaphosphoric acids, and the like. The polyphosphoric acid used in the present invention may be a mixture of the aforementioned polyphosphoric acids. Polyphosphoric acid can be prepared by, for example, (1) a method of heating H₃PO₄ to cause dehydration, or (2) a method of adding a sufficient amount of diphosphorus pentoxide (P₄O₁₀, phosphoric anhydride) to H₃PO₄. Since it is generally difficult to produce each polyphosphoric acid as a pure product, mixtures of polyphosphoric acid of various polymerization degrees are marketed. Polyphosphoric acid in the form of aqueous solution is easily hydrolyzed, and via polyphosphoric acid of lower molecular weight, eventually becomes orthophosphoric acid. The polymerization degree of the polyphosphoric acid used in the present invention is preferably 2 or higher, more preferably 5 or higher. The polymerization degree of the polyphosphoric acid used in the present invention is preferably 20 or lower, more preferably 12 or lower.

The polymerization degree of polyphosphoric acid is represented with (1) an analytical value obtained by acidimetry representing polymerization degree as a polymer of P₂O₅, or (2) a condensation ratio of polyphosphoric acid, i.e., a ratio of weight of orthophosphoric acid produced by hydrolysis of polyphosphoric acid with respect to the weight of the polyphosphoric acid, and in both cases, a larger value indicates a higher polymerization degree.

As the polyphosphoric acid used in the present invention, a marketed product can be used, and such a product has a polymerization degree as a polymer of P₂O₅ represented by an analytical value obtained in acidimetry of, for example, at least 76% or higher, preferably 81% or higher, more preferably 83% or higher. Alternatively, the polyphosphoric acid used in the present invention has a condensation ratio of polyphosphoric acid of at least 105% or higher, preferably 111% or higher, more preferably 115% or higher. A polyphosphoric acid having a condensation ratio of 85 to 120% is usually used.

A mixture of phosphoric acid and phosphoric anhydride (diphosphorus pentoxide) having a viscosity of 700 to 1000 mPa·s and a phosphoric acid concentration of 110 to 130% can also be used (Japanese Patent Unexamined Publication No. 2005-289949).

The polyphosphoric acid used in the present invention is, for example, a marketed polyphosphoric acid available from Riedel-de Haen.

### Heat treatment

As for the temperature of the heat treatment used in the present invention, it is desirable to perform the heat treat at such a temperature that the cyclization reaction quickly advances, and generation of impurities is minimized.

Therefore, the temperature of the heat treatment performed in the present invention is preferably a temperature near the boiling point of the organic solvent used.

In regard to this point, since the reaction mixture for the cyclization reaction is a mixture obtained by adding polyphosphoric acid and the organic solvent to be used to a compound of the formula (II) or (IV) before being cyclized, the temperature of the heat treatment performed in the present invention can be a temperature not lower than the boiling point of the organic solvent to be used.

Specifically, the temperature of the heat treatment performed in the present invention is preferably a temperature in the range of from minus 25°C to plus 25°C relative to the boiling point of the organic solvent used as the reference temperature. The boiling points of the organic solvents used in the present invention are 87°C for 1,1,2-trichloroethene (trichloroethylene), 101°C for methylcyclohexane, 81°C for cyclohexane, 98°C for n-heptane, 82°C for acetonitrile, 82°C for 1,2-dimethoxyethane, 101°C for 1,4-dioxane, 66°C for tetrahydrofuran, 153°C for N,N-dimethylformamide, 77°C for ethyl acetate, and 80°C for methyl ethyl ketone, and therefore, preferred temperature ranges for the heat treatment performed in the present invention are 62 to 112°C in the case of using 1,1,2-trichloroethene (trichloroethylene), 76 to 126°C in the case of using methylcyclohexane, 56 to 106°C in the case of using cyclohexane, 73 to 123°C in the case of using n-heptane, 57 to 107°C in the case of using acetonitrile, 57 to 107°C in the case of using 1,2-dimethoxyethane, 76 to 126°C in the case of using 1,4-dioxane, 41 to 91°C in the case of using tetrahydrofuran, 128 to 178°C in the case of using N,N-dimethylformamide, 52 to 102°C in the case of using ethyl acetate, and 55 to 105°C in the case of using methyl ethyl ketone.

Further, the temperature of the heat treatment suitable for the present invention is preferably a temperature in the range of from minus 20°C to plus 20°C relative to the boiling point of the organic solvent, more preferably a temperature in the range of from minus 15°C to plus 15°C relative to the boiling point of the organic solvent, still more preferably a temperature in the range of from minus 10°C to plus 10°C relative to the boiling point of the organic solvent, most preferably a temperature in the range of rom minus 5°C to plus 5°C relative to the boiling point of the organic solvent. As for these temperature ranges, preferred temperature ranges for each solvent are similarly determined according to the aforementioned ranges on the basis of the boiling point of each organic solvent.

If the time of the heat treatment performed in the present invention is too short, zaltoprofen or a zaltoprofen derivative is not sufficiently produced, and if the time is too long, amount of impurities increases.

Therefore, the time of the heat treatment performed in the present invention is desirably such a heat treatment time that a high yield is attained, and in addition, generation of impurities is minimized.

The time of the heat treatment performed in the present invention is preferably 1 to 8 hours, more preferably 3 to 6 hours, still more preferably 3.5 to 4.5 hours.

### Starting material

The starting material used in the present invention is a compound represented by following the formula (II): 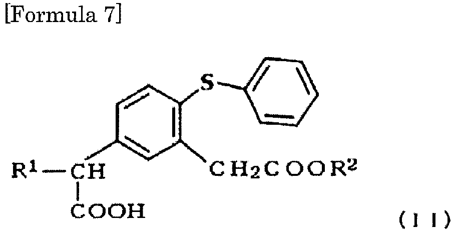 wherein, in the formula, R¹ and R² independently represent H or a hydrocarbon group having 1 to 8 carbon atoms, preferably independently represent H or a lower alkyl group having 1 to 4 carbon atoms, and more preferably R¹ represent methyl group, and and R² represents H or methyl group.

### Zaltoprofen or zaltoprofen derivative

In the present invention, zaltoprofen is a compound represented by following the formula (III).

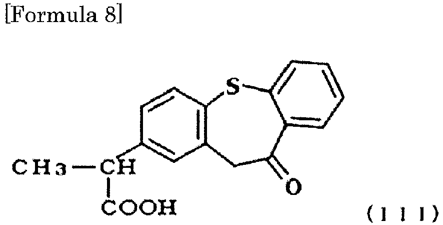

In the present invention, the zaltoprofen derivative is a compound represented by the following the formula (I): 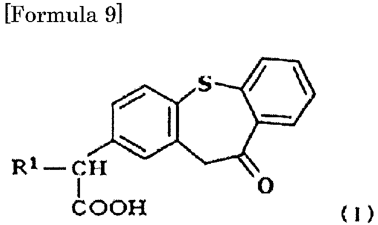 wherein, in the formula, R¹ is H or a hydrocarbon group having 2 to 8 carbon atoms. The hydrocarbon group having 2 to 8 carbon atoms mentioned above is preferably a lower alkyl having 2 to 8 carbon atoms, more preferably a lower alkyl having 2 to 4 carbon atoms.

### Extraction of zaltoprofen or zaltoprofen derivative

Yield of zaltoprofen or a zaltoprofen derivative prepared by the method for preparation of the present invention can be increased by extracting the product with an arbitrary organic solvent such as ethyl acetate. Further, the organic solvent containing zaltoprofen or a zaltoprofen derivative prepared by the method for preparation of the present invention can be dried over anhydrous sodium sulfate, or the like. Further, after a reaction for preparing an objective substance, or after drying an objective substance, recrystallization can generally be performed for the purpose of isolation and purification of the objective substance. The solvent suitable for such extraction and recrystallization is not particularly limited so long as the solvent dissolves the target substance. An optimal solvent can be used by choosing from water, methanol, ethanol, acetone, benzene, toluene, hexane, ethyl acetate, isopropyl ether, and a mixed solvent of these, which are generally usable in organic chemistry.

### Isolation and purification of zaltoprofen or zaltoprofen derivative

Although zaltoprofen or a zaltoprofen derivative prepared by the method for preparation of the present invention, per se, can be used as a medicament, insoluble matters may be removed from the product by adding an organic solvent such as acetone to the zaltoprofen or zaltoprofen derivative, dissolving the product therein by warming, and filtering the solution. Further, zaltoprofen or a zaltoprofen derivative prepared by the method for preparation of the present invention can also be deposited as crystals by adding an organic solvent such as acetone to the zaltoprofen or zaltoprofen derivative, dissolving the product therein by warming, and lowering the temperature.

It is also possible to further improve the method for preparation of the present invention by applying an arbitrary known technique. For example, since it is a generally empirically known fact that production scale and temperature may influence generation of crystals, objective crystals can be efficiently obtained by controlling production scale and temperature. Further, for example, it is frequently experienced that a one single compound exists in a plurality of different crystal forms, and it is known that, in such a case, a crystal form to be obtained may be chosen by temperature control such as setting of temperature, and cooling rate (PHARM TECH JAPAN, vol. 17, No. 12 (2001) pp.27-38, Japanese Patent Unexamined Publication No. 2006-160766). Further, it is also a well-known fact that objective crystals can generally be efficiently obtained by inoculating seed crystals.

### Examples

Hereafter, the present invention will be explained in more detail with reference to examples and comparative examples. However, the present invention is not limited by these.

### Example 1

### Preparation of zaltoprofen by cyclization performed in the presence of methylcyclohexane

To 5-(1-carboxyethyl)-2-phenylthiophenylacetic acid (50 g, 0.158 mol) represented by the following formula (IV): 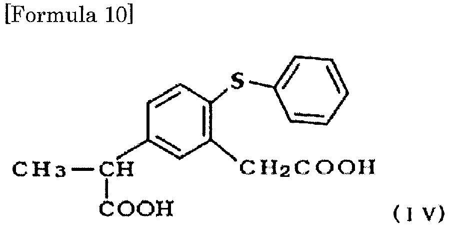 polyphosphoric acid (250 g; condensation degree, 105%) and methylcyclohexane (60 mL) were added, and with gradually warming the mixture, the mixture was stirred at an internal temperature of 90 to 95°C for 3.5 hours. Then, the warming was terminated, when the internal temperature became 45°C, water (250 mL) and ethyl acetate (250 mL) were simultaneously added dropwise to the reaction mixture, and the organic solvent layer was separated. The aqueous layer was extracted with ethyl acetate (150 mL), and ethyl acetate was separated and combined with the organic solvent layer obtained above, and washed by adding water (200 mL) and brine (200 mL), and separating the organic solvent layer. Anhydrous sodium sulfate (50 g) was added to the separated organic layer for dehydration, and the organic solvent was evaporated under reduced pressure to obtain an orange solid (54.7 g).

To this orange solid (54.7 g), acetone (100 mL) was added, and the solid was dissolved in acetone, the acetone was separated, and dried up. Acetone (240 mL) was added to the solid again, the solid was dissolved by warming at 50°C, and then the insoluble matter was separated by filtration. The resulting solution in acetone was evaporated by 1/2 volume, the remained solution (120 mL) was evaporated at room temperature for 22 hours with stirring under reduced pressure, and the deposited crystals were collected by filtration. The crystals obtained by the filtration were washed with cold acetone (20 mL), and then air-dried to obtain zaltoprofen (35.95 g, yield 76.2%) as white crystals. By high performance liquid chromatography, it was observed that these white crystals had a purity of 99.9%. As far as observation is made on the high performance liquid chromatography chart, any peaks other than the peak of zaltoprofen were not detected. As for the conditions of the high performance liquid chromatography, an ODS column produced by YMC Co., Ltd., A302 (internal diameter, 4.6 mm; column length, 15 cm) was used, the mobile phase of CH₃CN/H₂O/AcOH = 300:200:1 was used at a flow rate of 1 mL/min, and the detection was based on ultra violet absorbance at a wavelength of 240 nm. The unit of the retention time (R.T.) mentioned in the HPLC chart is minute (Fig. 1).

### Example 2

### Preparation of zaltoprofen by cyclization performed in the presence of n-heptane

To 5-(1-carboxyethyl)-2-phenylthiophenylacetic acid (100 g, 0.316 mol) represented by the following formula (IV): 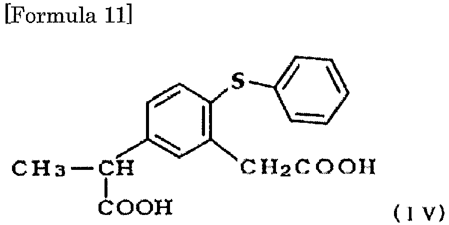 polyphosphoric acid (500 g; condensation degree, 105%) and n-heptane (120 mL) were added, and with gradually warming the mixture, the mixture was stirred at an internal temperature of 98°C for 4.5 hours. Then, the warming was terminated, when the internal temperature became 45°C, water (500 mL) and ethyl acetate (500 mL) were simultaneously added dropwise to the reaction mixture, so that the internal temperature did not exceed 60°C, and the organic solvent layer was separated. The aqueous layer was extracted with ethyl acetate (300 mL), and ethyl acetate was separated and combined with the organic solvent layer obtained above, and washed by adding water (500 mL) and brine (500 mL), and separating the organic solvent layer. Anhydrous sodium sulfate (50 g) was added to the separated organic layer for dehydration, and then the organic solvent was evaporated under reduced pressure to obtain a solid (89.9 g).

To this solid (89.9 g), acetone (450 mL) was added, the solid was dissolved in acetone on a water bath at 50°C, and then the insoluble matter was separated by filtration. The resulting solution in acetone was evaporated by 1/2 volume, the remained solution was stirred overnight at room temperature, and then the deposited crystals were collected by filtration. The crystals obtained by the filtration were washed with acetone, and then air-dried to obtain zaltoprofen (64.7 g, yield 72.0%) as white crystals. By high performance liquid chromatography performed under the same conditions as those of Example 1, it was observed that these white crystals had a purity of 99.8%. As far as observation is made on the high performance liquid chromatography chart, any peaks other than the peak of zaltoprofen were not detected (Fig. 2).

### Example 3

### Preparation of zaltoprofen by cyclization performed in the presence of 1,1,2-trichloroethene

To 5-(1-carboxyethyl)-2-phenylthiophenylacetic acid (5.20 g, 16.4 mmol) represented by the following formula (IV): 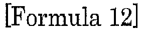 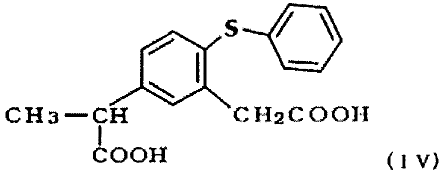 polyphosphoric acid (33.5 g; condensation degree, 105%) and 1,1,2-trichloroethene (8.0 mL) were added, and with gradually warming the mixture, the mixture was stirred at an internal temperature of 89°C for 3 hours. Then, the warming was terminated, when the internal temperature became 40°C, water (25 mL) and ethyl acetate (25 mL) were simultaneously added dropwise to the reaction mixture so that the internal temperature did not exceed 60°C, and the organic solvent layer was separated. The aqueous layer was extracted with ethyl acetate (15 mL), and the extracted ethyl acetate was combined with the aforementioned organic solvent layer to obtain a zaltoprofen fraction. The zaltoprofen fraction was washed by adding water (30 mL) and brine (30 mL), and separating the organic solvent layer. Anhydrous sodium sulfate (5 g) was added to the separated organic layer for dehydration, and the organic solvent was evaporated under reduced pressure to obtain a solid (4.8 g).

To this solid (4.8 g), acetone (25 mL) was added, the solid was dissolved in acetone on a water bath at 50°C, and then the insoluble matter was separated by filtration. The resulting solution in acetone was evaporated by 1/2 volume, the remained solution was stirred overnight at room temperature, and then the deposited crystals were collected by filtration. The crystals obtained by the filtration were washed with acetone (5 mL), and then air-dried to obtain zaltoprofen (3.20 g, yield 65.3%) as white crystals. By high performance liquid chromatography performed under the same conditions as those of Example 1, it was observed that these white crystals had a purity of 99.9%. As far as observation is made on the high performance liquid chromatography chart, any peaks other than the peak of zaltoprofen were not detected (Fig. 3).

### Example 4

### Example of cyclization performed in the presence of cyclohexane

Zaltoprofen was prepared by cyclizing the compound of the formula (IV) in the same manner as that of Example 1, and purity thereof was observed by HPLC performed under the same conditions as those of Example 1, provided that cyclohexane was used as the solvent for the cyclization, the heat treatment temperature for the cyclization was 95 to 100°C, and the heat treatment time for the cyclization was 1 hour. By high performance liquid chromatography performed under the same conditions as those of Example 1, it was observed that zaltoprofen having a purity of 99.8% was finally obtained as white crystals (yield 63.7%). Further, as far as observation is made on the high performance liquid chromatography chart, any peaks other than the peak of zaltoprofen were not detected (Fig. 4).

### Example 5

### Example of cyclization performed in the presence of acetonitrile (CH₃CN)

Zaltoprofen was prepared by cyclizing the compound of the formula (IV) in the same manner as that of Example 1, provided that acetonitrile was used as the organic solvent for the cyclization, the heat treatment temperature for the cyclization was 100°C, and the heat treatment time for the cyclization was 1 hour. Purity of the product was measured by thin layer chromatography (CHCl₃:MeOH:AcOH = 100:10:1, detection was based on ultraviolet absorption at a wavelength of 254 nm), and production of zaltoprofen was confirmed. There were also observed unreacted compound of the formula (IV), and a small amount of impurity as a spot near the starting point (Fig. 5).

### Example 6

### Example of cyclization performed in the presence of 1,2-dimethoxyethane

Zaltoprofen was prepared by cyclizing the compound of the formula (IV) in the same manner as that of Example 1, provided that 1,2-dimethoxyethane was used as the organic solvent for the cyclization, the heat treatment temperature for the cyclization was 95 to 100°C, and the heat treatment time for the cyclization was 5 hours. Purity of the product was measured by thin layer chromatography (CHCl₃:MeOH:AcOH = 100:10:1, detection was based on ultraviolet absorption at a wavelength of 254 nm), and production of zaltoprofen was confirmed as a major spot. There were also observed unreacted compound of the formula (IV), a small amount of impurity (spot near the starting point), and unknown impurity (spot near the solvent front) (Fig. 6).

### Example 7

### Example of cyclization performed in the presence of 1,4-dioxane

Zaltoprofen was prepared by cyclizing the compound of the formula (IV) in the same manner as that of Example 1, provided that 1,4-dioxane was used as the organic solvent for the cyclization, the heat treatment temperature for the cyclization was 100 to 115°C, and the heat treatment time for the cyclization was 5 hours. By detection based on thin layer chromatography (CHCl₃:MeOH:AcOH = 100:10:1, detection was based on ultraviolet absorption at a wavelength of 254 nm), production of a small amount of zaltoprofen was observed. Presence of a large amount of unreacted compound of the formula (IV) was also observed.

### Example 8

### Example of cyclization performed in the presence of tetrahydrofuran

Zaltoprofen was prepared by cyclizing the compound of the formula (IV) in the same manner as that of Example 1, provided that tetrahydrofuran was used as the organic solvent for the cyclization, the heat treatment temperature for the cyclization was 100°C, and the heat treatment time for the cyclization was 5 hours. Purity of the product was measured by thin layer chromatography (CHCl₃:MeOH:AcOH = 100:10:1, detection was based on ultraviolet absorption at a wavelength of 254 nm), and production of zaltoprofen was clearly confirmed. There were also observed unreacted compound of the formula (IV), and a small amount of impurity (spot near the starting point) (Fig. 7).

### Example 9

### Example of cyclization performed in the presence of N,N-dimethylformamide

Zaltoprofen was prepared by cyclizing the compound of the formula (IV) in the same manner as that of Example 1, provided that N,N-dimethylformamide was used as the organic solvent for the cyclization, the heat treatment temperature for the cyclization was 115°C, and the heat treatment time for the cyclization was 6 hours. Purity of the product was measured by thin layer chromatography (CHCl₃:MeOH:AcOH = 100:10:1, detection was based on ultraviolet absorption at a wavelength of 254 nm), and as a result, almost no production of zaltoprofen was observed.

### Example 10

### Example of cyclization performed in the presence of ethyl acetate

Zaltoprofen was prepared by cyclizing the compound of the formula (IV) in the same manner as that of Example 1, provided that ethyl acetate was used as the organic solvent for the cyclization, the heat treatment temperature for the cyclization was 100°C, and the heat treatment time for the cyclization was 6 hours. Purity of the product was measured by thin layer chromatography (CHCl₃:MeOH:AcOH = 100:10:1, detection was based on ultraviolet absorption at a wavelength of 254 nm), and as a result, it was observed that zaltoprofen was a major product, and there were also observed unreacted compound of the formula (IV), and a small amount of impurity considered to be a decomposition product (spot near the solvent front) (Fig. 8).

### Example 11

### Example of cyclization performed in the presence of methyl ethyl ketone

Zaltoprofen was prepared by cyclizing the compound of the formula (IV) in the same manner as that of Example 1, provided that methyl ethyl ketone was used as the organic solvent for the cyclization, the heat treatment temperature for the cyclization was 95 to 101°C, and the heat treatment time for the cyclization was 2 hours. Purity of the product was measured by thin layer chromatography (CHCl₃:MeOH:AcOH = 100:10:1, detection was based on ultraviolet absorption at a wavelength of 254 nm), and as a result, production of zaltoprofen was observed, and there were also observed unreacted compound of the formula (IV), and four kinds of impurities considered to be decomposition products (spots between the spot of zaltoprofen and the solvent front) (Fig. 9).

### Comparative Example 1

### Example of cyclization performed in the presence of xylene

Zaltoprofen was prepared by cyclizing the compound of the formula (IV) in the same manner as that of Example 1, provided that xylene was used as the organic solvent for the cyclization, the heat treatment temperature for the cyclization was 97 to 100°C, and the heat treatment time for the cyclization was 2 hours. By high performance liquid chromatography performed under the same conditions as those of Example 1, it was observed that zaltoprofen having a purity of 99.8% was finally obtained as white crystals (yield 57.8%). Further, as far as observation is made on the high performance liquid chromatography chart, any peaks other than the peak of zaltoprofen were not detected (Fig. 10).

### Comparative Example 2

### Example of cyclization performed in the presence of 1,2-dichloroethane

Zaltoprofen was prepared by cyclizing the compound of the formula (IV) in the same manner as that of Example 1, and purity of the product was measured by HPLC performed under the same conditions as those of Example 1, provided that 1,2-dichloroethane was used as the organic solvent for the cyclization, the heat treatment temperature for the cyclization was 85 to 95°C, and the heat treatment time for the cyclization was 4.5 hours. By high performance liquid chromatography performed under the same conditions as those of Example 1, it was observed that zaltoprofen having a purity of 99.9% was finally obtained as white crystals (yield 67.2%). Further, as far as observation is made on the high performance liquid chromatography chart, any peaks other than the peak of zaltoprofen were not detected (Fig. 11). Comparative Example 3

### Example of cyclization performed in the presence of toluene

Zaltoprofen was prepared by cyclizing the compound of the formula (IV) in the same manner as that of Example 1, and purity of the product was measured by HPLC performed under the same conditions as those of Example 1, provided that toluene was used as the organic solvent for the cyclization, the heat treatment temperature for the cyclization was 97°C, and the heat treatment time for the cyclization was 3 hours. By high performance liquid chromatography performed under the same conditions as those of Example 1, it was observed that zaltoprofen having a purity of 99.1% was finally obtained as pale yellow crystals (Fig. 12).

### Comparative Example 4

### Example of cyclization performed in the presence of chloroform

Zaltoprofen was prepared by cyclizing the compound of the formula (IV) in the same manner as that of Example 1, and purity of the product was measured by HPLC performed under the same conditions as those of Example 1, provided that chloroform was used as the organic solvent for the cyclization, the heat treatment temperature for the cyclization was 98°C, and the heat treatment time for the cyclization was 4 hours. By high performance liquid chromatography performed under the same conditions as those of Example 1, it was observed that zaltoprofen having a purity of 99.5% was finally obtained as white crystals (Fig. 13).

### Industrial Applicability

According to the method for preparation of the present invention, zaltoprofen or a zaltoprofen derivative of a purity not lower than 99% can be prepared in a yield not lower than 60% by using polyphosphoric acid in an amount of 6.5 times or less of the amount of a compound of the formula (II) or (IV) as a condensing agent. Therefore, the method is industrially useful.

According to the method for preparation of the present invention, zaltoprofen or a zaltoprofen derivative can be prepared by using an organic solvent that hardly imposes concerns about health hazard for workers on the manufacturing site, and is easily handled in operations. Therefore, the method is suitable for industrial production.

According to the method for preparation of the present invention, zaltoprofen or a zaltoprofen derivative can be prepared on an industrial scale in a simple and economical manner. Therefore, practical application thereof is greatly expected for industrial manufacture of zaltoprofen, which is used for anti-inflammatory and analgesic purposes in many countries and areas.

## Claims

1. A method for preparing a compound represented by the following formula (I): 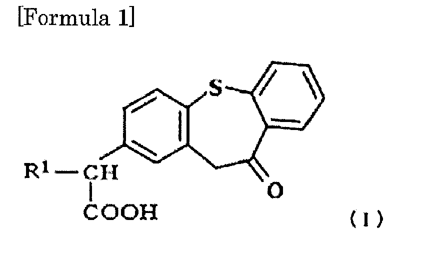 wherein, in the formula (I);
R¹ is H or a hydrocarbon group having 1 to 8 carbon atoms, which comprises cyclizing a compound represented by the following formula (II): 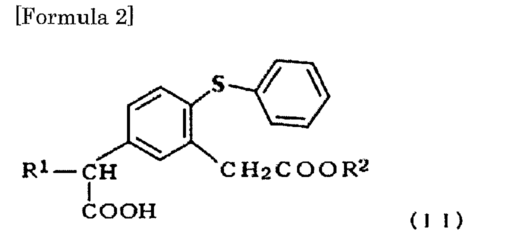
wherein, in the formula (II),
R¹ is H or a hydrocarbon group having 1 to 8 carbon atoms; and
R² is H or a hydrocarbon group having 1 to 8 carbon atoms;
by subjecting the compound of the formula (II) to a heat treatment together with at least one kind of condensing agent selected from the group consisting of polyphosphoric acid, orthophosphoric acid, and concentrated sulfuric acid,
in the presence of at least one kind of organic solvent selected from the group consisting of 1,1,2-trichloroethene, methylcyclohexane, cyclohexane, n-heptane, acetonitrile, 1,2-dimethoxyethane, 1,4-dioxane, tetrahydrofuran, N,N-dimethylformamide, ethyl acetate, and methyl ethyl ketone.

2. The method for preparation according to claim 1, wherein the organic solvent is at least one kind of organic solvent selected from the group consisting of 1,1,2-trichloroethene, methylcyclohexane, cyclohexane, and n-heptane.

3. The method for preparation according to claim 1 or 2, wherein the condensing agent is polyphosphoric acid.

4. The method for preparation according to any one of claims 1 to 3, wherein the heat treatment is performed at a temperature in the range of from minus 25°C to plus 25°C relative to the boiling point of the organic solvent used as the reference.

5. The method for preparation according to any one of claims 1 to 4, wherein time of the heat treatment is 1 to 8 hours.

6. The method for preparation according to any one of claims 1 to 5, wherein weight of the condensing agent is 1 to 10 times of the weight of the compound represented by the formula (II).

7. The method for preparation according to any one of claims 1 to 6, wherein volume of the organic solvent is 50 to 100 mL per 50 g of the compound represented by the formula (II).

8. The method for preparation according to any one of claims 1 to 7, wherein R¹ is methyl group.

9. The method for preparation according to any one of claims 1 to 8, wherein R² is H or methyl group.

10. The method for preparation according to any one of claims 1 to 9, wherein polyphosphoric acid having a condensation ratio of 105% or higher is used.

11. The method for preparation according to any one of claims 1 to 10, which further comprises extracting zaltoprofen or zaltoprofen derivative by using ethyl acetate.

12. The method for preparation according to any one of claims 1 to 11, which further comprises drying zaltoprofen or zaltoprofen derivative over anhydrous sodium sulfate.

13. The method for preparation according to any one of claims 1 to 12, which further comprises adding an organic solvent to zaltoprofen or zaltoprofen derivative, dissolving zaltoprofen or zaltoprofen derivative by warming, and removing insoluble matter by filtration.

14. The method for preparation according to any one of claims 1 to 13, which further comprises adding an organic solvent to zaltoprofen or zaltoprofen derivative, dissolving zaltoprofen or zaltoprofen derivative by warming, and then crystallizing zaltoprofen or zaltoprofen derivative by lowering the temperature.
